# EUROPEAN PATENT SPECIFICATION

(11) **EP 3 434 122 B1**
(45) Date of publication and mention of the grant of the patent: **22.03.2023**
(21) Application number: 16914942.4
(22) Date of filing: 16.12.2016
(51) Int. Cl.: A24F 40/05, A24F 40/44, A24F 40/485, A61M 15/00, A61M 15/06

(54) **ULTRASONIC ELECTRONIC CIGARETTE ATOMIZING CORE**
ZERSTÄUBUNGSKERN EINER ELEKTRONISCHEN ULTRASCHALLZIGARETTE
NOYAU D'ATOMISATION DE CIGARETTE ÉLECTRONIQUE PAR ULTRASONS

(30) Priority: 31.08.2016 CN 201621016377 U; 31.08.2016 CN 201621014101 U
(43) Date of publication of application: 30.01.2019
(73) Proprietor: China Tobacco Hunan Industrial Co., Ltd., Changsha, Hunan 410007 (CN)
(72) Inventor: LIU, Jianfu, Changsha Hunan 410007 (CN); ZHONG, Kejun, Changsha Hunan 410007 (CN); GUO, Xiaoyi, Changsha Hunan 410007 (CN); HUANG, Wei, Changsha Hunan 410007 (CN); YU, Hong, Changsha Hunan 410007 (CN); DAI, Yuangang, Changsha Hunan 410007 (CN); YIN, Xinqiang, Changsha Hunan 410007 (CN); YI, Jianhua, Changsha Hunan 410007 (CN); SHEN,Lizhou, Changsha Hunan 410007 (CN)
(74) Representative: Kramer Barske Schmidtchen Patentanwälte PartG mbB
(86) International application number: PCT/CN2016/110389
(87) International publication number: WO 2018/040380

(56) References cited:
- CN-A- 101 878 958
- CN-A- 105 795 526
- CN-A- 105 795 527
- CN-U- 202 286 306
- CN-U- 203 424 288
- CN-U- 203 424 288
- CN-U- 203 662 023
- CN-U- 205 378 853
- CN-U- 205 432 145
- US-A1- 2014 150 783
- US-A1- 2014 311 503

## Description

### Field of the Invention

The present invention belongs to the technical field of electronic cigarette, and in particular to an ultrasonic electronic cigarette atomization core.

### Background of the Invention

At present, atomization cotton in the existing high-frequency ultrasonic electronic cigarette atomizers is of a piece-shaped structure, the atomization cotton is laminated on an upper surface of an atomization piece, and both of the atomization cotton and the atomization piece are vertical to the length direction of the electronic cigarette. There are two air path directions at present, one air path direction is that the air enters from top to bottom of the atomization cotton, turns to the center of the atomization cotton after arriving at the edge of the upper surface of the piece-shaped atomization cotton, and outflows upward after taking away the smoke at the center of the atomization cotton; and the other air path direction is that the air enters from bottom to top of the atomization piece, and the airflow outflows from the top after coming round to the surface of the atomization cotton from the bottom surface of the atomization piece. Both of the two air path directions have the following defects: first, as the airflow only passes through the surface of the atomization cotton, the airflow can only take away the smoke on the surface of the atomization cotton but cannot take away the smoke that is stored inside the atomization cotton and not automatically ejected out, namely cannot take away all the smoke produced by atomization, and thus the amount of the smoke is small. Second, the airflow directly takes large-granule smoke produced by ultrasonic vibration atomization into the mouth, resulting in a poor user experience.

In addition, the tobacco tar guide modes of the existing ultrasonic electronic cigarette atomization cores all use one-stage tobacco tar guide structures, even if the tobacco tar is directly guided from a tobacco tar bin by using jaconet, as no tobacco tar storage cotton used for buffering the tobacco tar is provided, the supply speed of tobacco tar cannot be controlled, and it is prone to cause the phenomenon of tobacco tar submersion or insufficient tobacco tar supply.

CN 205 432 145 U discloses an ultrasonic nebulizer and an electronic cigarette. CN 203 662 023 U discloses a supersonic atomizer. CN 202 286 306 U discloses a unit atomized cartridge for an electronic cigarette.

### Summary of the Invention

An objective of the present invention is to provide an ultrasonic electronic cigarette atomization core in view of the shortcomings of the above prior art. The air path structure and the tobacco tar guide structure of the atomization core are improved, a larger amount of smoke can be produced, the taste of smoke is good, and the phenomenon of tobacco tar submersion or insufficient tobacco tar supply is unlikely to occur.

In order to solve the above technical problem, the present invention adopts the technical solution as follows:
An ultrasonic electronic cigarette atomization core includes an atomization core sleeve, an atomization piece is provided in the atomization core sleeve, the ultrasonic electronic cigarette atomization core is structurally characterized in that an air passage, an air pass pipe and a cup-shaped tobacco tar guide cotton are further provided in the atomization core sleeve, an outer bottom surface of the tobacco tar guide cotton is in contact with a top surface of the atomization piece, and a side wall of the tobacco tar guide cotton is sheathed at the outside of the bottom of the side wall of the air pass pipe; the airflow enters from the air passage, flows by an inner bottom surface of the tobacco tar guide cotton after penetrating through the bottom of the side wall of the tobacco tar guide cotton, and outflows from the air pass pipe; or the airflow enters from a top end of the air pass pipe, penetrates through the bottom of the side wall of the tobacco tar guide cotton after flowing by the tobacco tar guide cotton, and outflows from the air passage.

By means of the above structure, the airflow enters from the air passage, enters the central position of the inner bottom surface of the tobacco tar guide cotton after penetrating through the side wall of the tobacco tar guide cotton, and then sucked out; or the airflow enters from the top of the air pass pipe, directly rushes to the central atomization area of the tobacco tar guide cotton and outflows from the air passage after penetrating through the side wall of the tobacco tar guide cotton. According to the air pass mode in the present invention, as the airflow penetrates through the side wall of the tobacco tar guide cotton, the smoke that is stored in the tobacco tar guide cotton and not automatically ejected out can be taken away, and thus a smoke amount larger than that of the traditional structure is produced.

If the air pass pipe is used as an air inlet pipe, the airflow in the air pass pipe can also flush large-granule smoke produced by ultrasonic vibration back to the surface of the atomization piece to be atomized again, and the small-granule smoke is taken out from the air passage after penetrating through the side wall of the tobacco tar guide cotton, therefore the taste of the smoke is better and smoother.

As a preferred embodiment, the atomization core sleeve includes an atomization core outer sleeve and an atomization core inner sleeve sheathed in the atomization core outer sleeve, a gap is provided between the top end of an inner side wall of the atomization core inner sleeve and an outer side wall of the air pass pipe, an air pass groove which communicates with the bottom of the side wall of the tobacco tar guide cotton is provided in a bottom end of the outer side wall of the atomization core inner sleeve, and a first through hole which communicates the gap with the air pass groove is provided in the side wall of the atomization core inner sleeve.

By means of the above structure, the gap, the first through hole, the air pass groove, the bottom of the side wall of the tobacco tar guide cotton and the air pass pipe communicate with each other in sequence to form an air path.

As another preferred embodiment, the atomization core sleeve includes an atomization core outer sleeve and an atomization core inner sleeve sheathed in the atomization core outer sleeve, and a second through hole which is located in the above-below direction and communicates with the bottom of the side wall of the tobacco tar guide cotton is provided in the side wall of the atomization core inner sleeve.

By means of the above structure, the second through hole, the bottom of the side wall of the tobacco tar guide cotton and the air pass pipe communicate with each other in sequence to form the air path. In such a structure, a hole is directly drilled in the side wall of the atomization core inner sleeve to allow the air to pass. Compared with the manner of providing the air pass groove and the first through hole in the side wall of the atomization core inner sleeve, the atomization core inner sleeve in this structure is relatively simpler to manufacture, and the sealing performance of the air path is more reliable.

Further, tobacco tar storage cotton sheathed at the outside of the side wall of the tobacco tar guide cotton is further provided in the atomization core sleeve, and a tobacco tar inlet hole which injects tobacco tar to the tobacco tar storage cotton is provided in the side wall of the atomization core sleeve.

A tobacco tar guide structure, in which the tobacco tar storage cotton and the tobacco tar guide cotton are matched with each other, is adopted to buffer the tobacco tar in the tobacco tar storage cotton, the tobacco tar storage cotton can ensure timely tobacco tar supply, and moreover, as the tobacco tar guide cotton has certain tobacco tar locking performance, the supply speed of tobacco tar is controllable, and the problem that the atomization piece is soaked in the tobacco tar caused by the over fast tobacco tar supply can be prevented; and by means of the tobacco tar storage performance of the tobacco tar storage cotton, the tobacco tar on the surface of the atomization piece can be supplemented quickly, therefore the phenomenon of insufficient tobacco tar supply caused by continuous suction can be well solved, and the phenomenon of dry burning or small smoke amount is avoided.

Further, a third through hole corresponding to the central position of the atomization piece is provided in the tobacco tar guide cotton.

The third through hole is provided in the center of the tobacco tar guide cotton, the central position (where the temperature is the highest) of the atomization piece is not blocked by the tobacco tar guide cotton, therefore the tobacco tar can be atomized instantly, and the smoke is generated instantaneously, thereby accelerating the atomization speed and reducing the phenomenon of tobacco tar submersion.

Further, the ultrasonic electronic cigarette atomization core further includes an elastic bracket, and a step part is provided on the inner side wall of the air pass pipe; and the bottom end of the elastic bracket props against the inner bottom surface of the tobacco tar guide cotton, and the top end of the elastic bracket props against the step part.

As the elastic bracket presses down the inner bottom surface of the tobacco tar guide cotton, the problem that atomization effect is affected caused by the deformation of the bottom surface of the tobacco tar guide cotton during inhalation can be prevented.

Further, an ultrasonic electronic cigarette atomizer is provided, including a tobacco tar bin, and the ultrasonic electronic cigarette atomization core is provided in the tobacco tar bin.

Further, the ultrasonic electronic cigarette atomizer further includes a suction nozzle located at the top of the tobacco tar bin and a first air inlet hole which communicates with the air passage, and the top end of the air pass pipe communicates with the suction nozzle.

By means of the above structure, the airflow enters from the first air inlet hole, flows by the air passage, penetrates through the bottom of the side wall of the tobacco tar guide cotton, and outflows from the suction nozzle through the inner bottom surface of the tobacco tar guide cotton and the air pass pipe.

Further, an airflow baffle vertical to the axial line of the air pass pipe is further provided above the air pass pipe, the air pass pipe aligns to the central position of the airflow baffle, a fourth through hole deviated from the central position of the airflow baffle is provided in the airflow baffle, and the air pass pipe communicates with the suction nozzle through the third through hole.

By means of the above structure, the airflow flows from the air pass pipe to the central position of the airflow baffle, turns around and enters the suction nozzle from the fourth through hole. The large-granule smoke is condensed on the central position of the airflow baffle after being collided so as not to be inhaled into the oral cavity by the human body, the taste of the smoke is better and smoother, and the user experience is good.

Further, the ultrasonic electronic cigarette atomizer further includes a suction nozzle base which connects the side wall of the tobacco tar bin with the suction nozzle, the first air inlet hole is provided in the side wall of the suction nozzle base, and an air adjusting ring corresponding to the first air inlet hole in position is further provided at the outside of the side wall of the suction nozzle base.

According to individual preference, the actual opening size of the first air inlet hole of the air adjusting ring can be adjusted to adjust the amount of the airflow entering the first air inlet hole.

Further, the ultrasonic electronic cigarette atomizer further includes a suction nozzle located at the top of the tobacco tar bin, an air inlet passage which communicates with the air pass pipe, and an air outlet hole which communicates the suction nozzle with the air passage.

By means of the above structure, the airflow enters from the air inlet passage, flows by the air pass pipe, penetrates through the bottom of the side wall of the tobacco tar guide cotton and outflows from the suction nozzle through the air passage and the air outlet hole.

Further, the ultrasonic electronic cigarette atomizer further includes a suction nozzle base which connects the side wall of the tobacco tar bin with the suction nozzle, an air inlet ring is provided in the suction nozzle base, the first air inlet hole is provided in the side wall of the suction nozzle base, a second air inlet hole which communicates the first air inlet hole with the air pass pipe is provided in the air inlet ring, and the air outlet hole is provided in the air inlet ring.

By means of the above structure, the airflow enters from the first air inlet hole, flows by the second air inlet hole and the air pass pipe in sequence, penetrates through the bottom of the side wall of the tobacco tar guide cotton and outflows from the suction nozzle through the air passage and the air outlet hole.

Further, an air adjusting ring corresponding to the position of the first air inlet hole is further provided in the side wall of the suction nozzle base.

According to individual preference, the actual opening size of the first air inlet hole of the air adjusting ring can be adjusted to adjust the amount of the airflow entering the first air inlet hole.

Further, the ultrasonic electronic cigarette atomizer further includes a base; an upper end cover is connected between the top of the side wall of the tobacco tar bin and the side wall of the suction nozzle base, a lower end cover is connected to the bottom of the side wall of the tobacco tar bin, the top of the atomization core sleeve is connected with the upper end cover through a connecting pipe, and the bottom of the atomization core sleeve is in threaded connection with the top of the side wall of the lower end cover; and the side wall of the base is in threaded connection with the bottom of the side wall of the lower end cover.

By means of the above structure, the atomization core can be independently replaced and is convenient to detach and install. As the atomization core is fixed to the tobacco tar bin by threads, when the base is unscrewed from the lower end cover, direct effusion of the tobacco tar can be avoided by the blockage of the atomization core sleeve, thereby being clean and sanitary.

Compared with the prior art, the present invention has the advantages of larger amount of smoke, better taste of the smoke, stable and reliable supply speed of tobacco tar, the phenomenon of tobacco tar submersion or insufficient tobacco tar supply is unlikely to occur, and the tobacco tar will not directly effuse when the base is unscrewed, thereby being clean and sanitary.

### Brief Description of the Drawings

Fig.1 is a main section view of embodiment 1 of an atomization core.
Fig.2 is a side section view of embodiment 1 of the atomization core.
Fig.3 is a main section view of embodiment 1 of an atomizer.
Fig.4 is an oblique section view of embodiment 1 of the atomizer.
Fig.5 is a main section view of embodiment 2 of the atomization core.
Fig.6 is a side section view of embodiment 2 of the atomization core.
Fig.7 is a main section view of embodiment 2 of the atomizer.
Fig.8 is a side section view of embodiment 2 of the atomizer.
Fig.9 is a main section view of embodiment 3 of the atomization core.
Fig.10 is a main section view of embodiment 3 of the atomizer.
Fig.11 is a side section view of embodiment 3 of the atomizer.

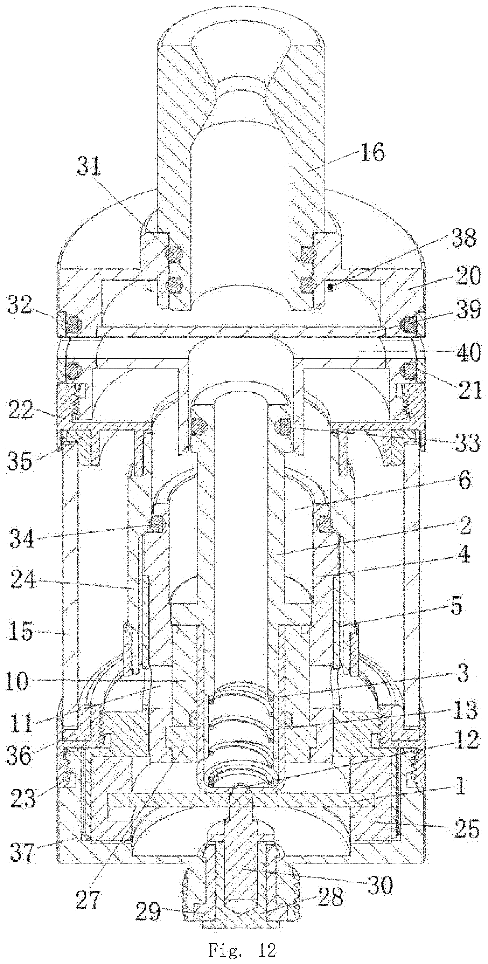

Fig.12 is an oblique section view of embodiment 3 of the atomizer.

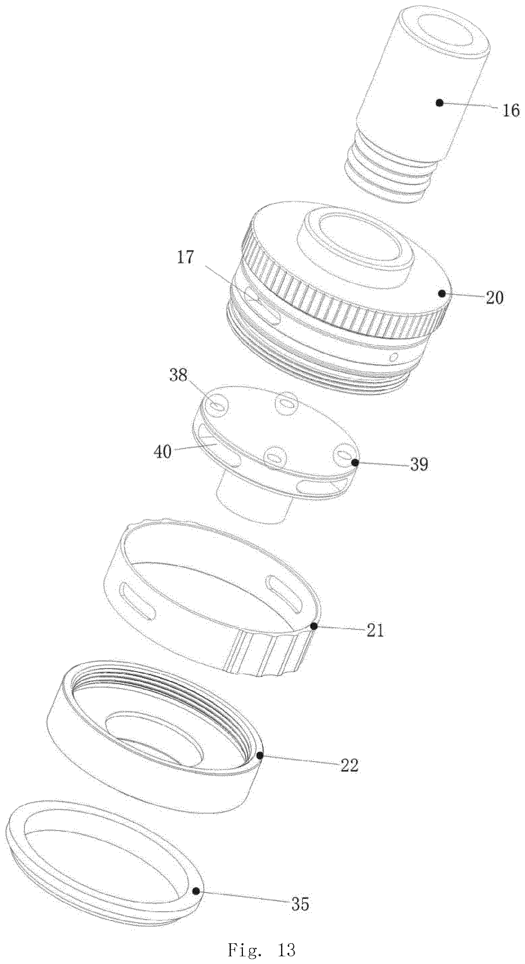

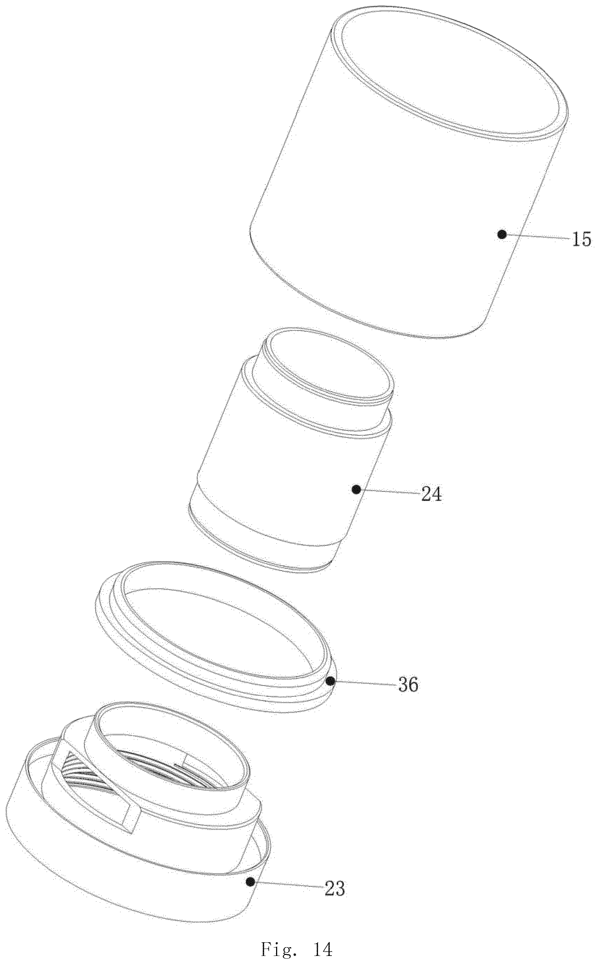

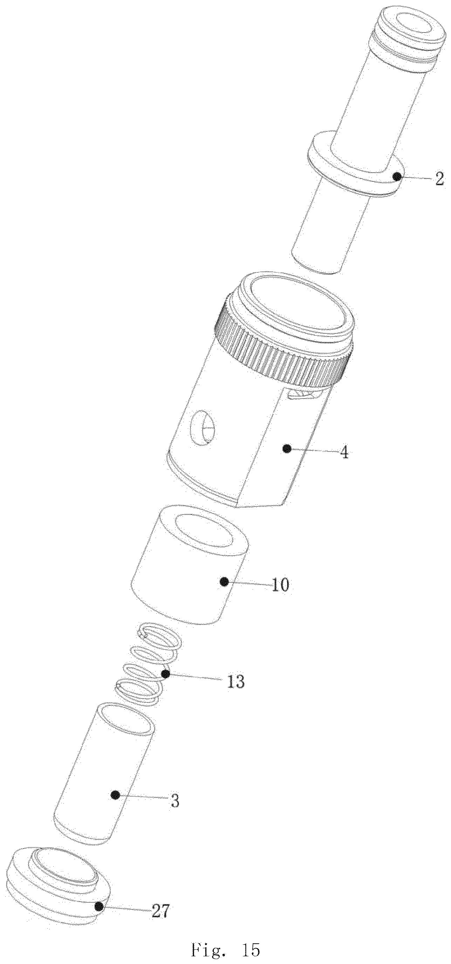

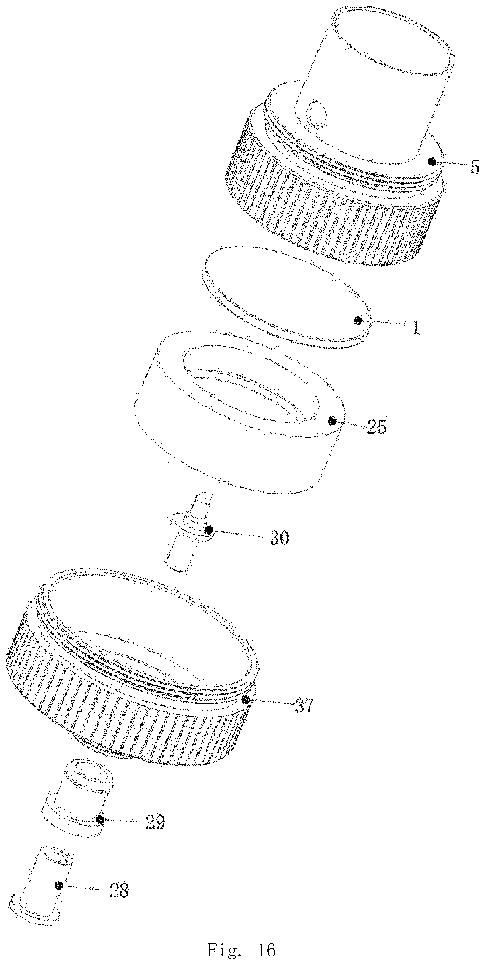

Fig.13 to Fig. 16 are explosive views of embodiment 3 of the atomizer.

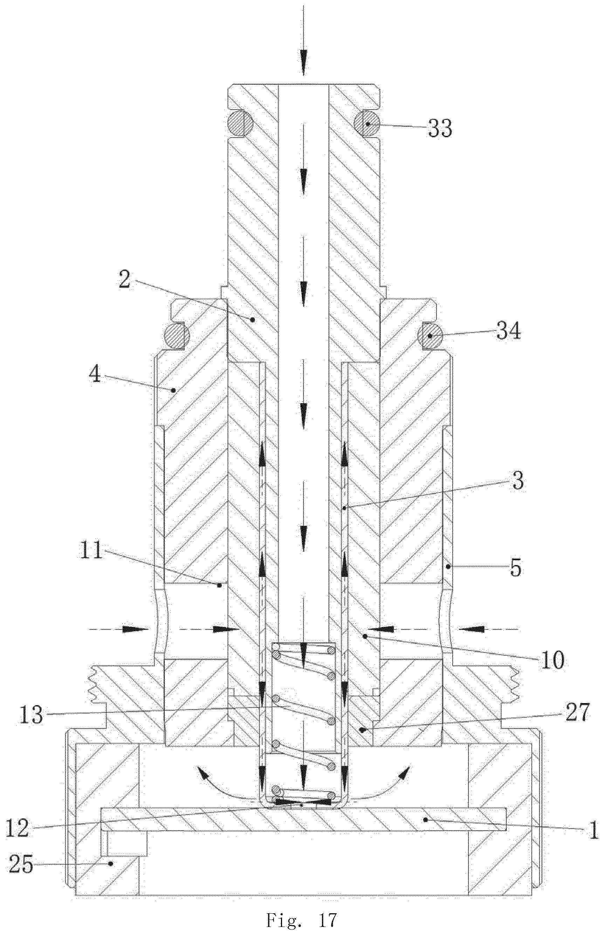

Fig.17 is a main section view of embodiment 4 of the atomization core.

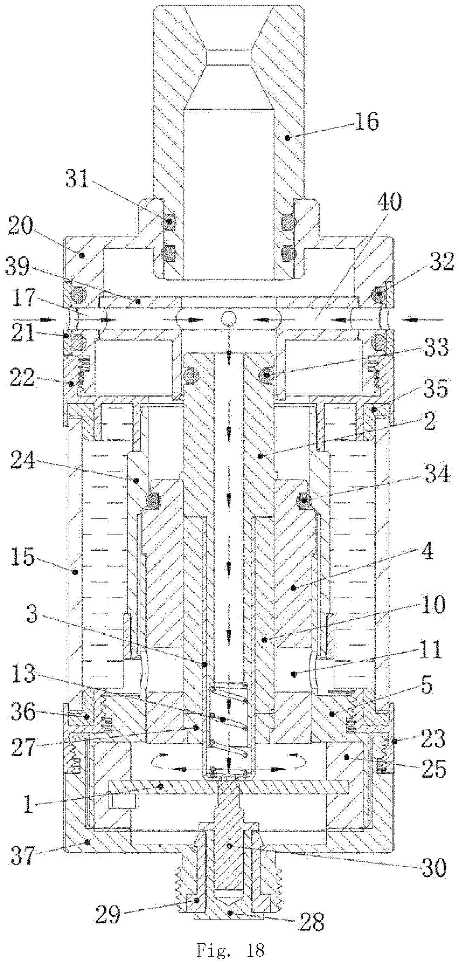

Fig.18 is a main section view of embodiment 4 of the atomizer.

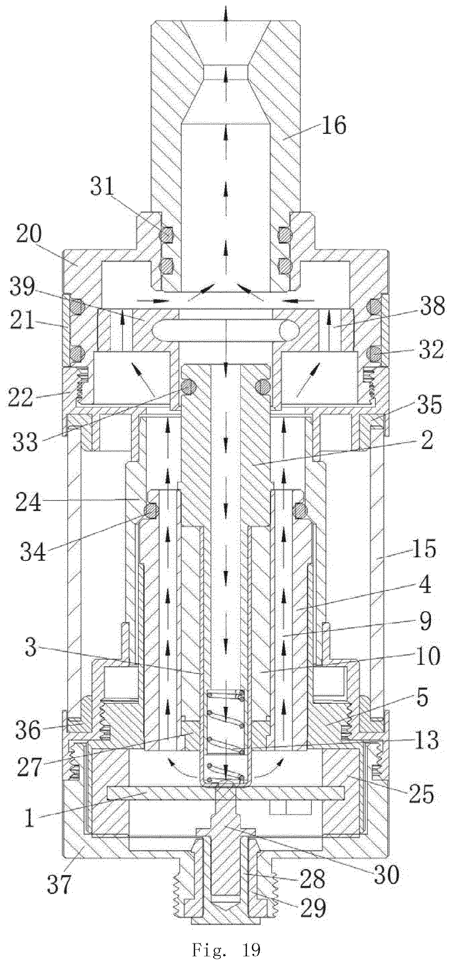

Fig.19 is a side section view of embodiment 4 of the atomizer.

In the figures, 1 represents an atomization piece, 2 represents an air pass pipe, 3 represents tobacco tar guide cotton, 4 represents an atomization core inner sleeve, 5 represents an atomization core outer sleeve, 6 represents a gap, 7 represents an air pass groove, 8 represents a first through hole, 9 represents a second through hole, 10 represents tobacco tar storage cotton, 11 represents a tobacco tar inlet hole, 12 represents a third through hole, 13 represents an elastic bracket, 14 represents a step part, 15 represents a tobacco tar bin, 16 represents a suction nozzle, 17 represents a first air inlet hole, 18 represents an airflow baffle, 19 represents a fourth through hole, 20 represents a suction nozzle base, 21 represents an air adjusting ring, 22 represents an upper end cover, 23 represents a lower end cover, 24 represents a connecting pipe, 25 represents a silica gel base, 26 represents a connecting joint, 27 represents a silica gel plug, 28 represents an electrode ring, 29 represents an insulating ring, 30 represents a spring plunger, 31 represents a first sealing ring, 32 represents a second sealing ring, 33 represents a third sealing ring, 34 represents a fourth sealing ring, 35 represents an upper sealing ring, 36 represents a lower sealing ring, 37 represents a base, 38 represents an air outlet hole, 39 represents an air inlet ring, and 40 represents a second air inlet hole.

### Detailed Description of the Embodiments

### Embodiment 1

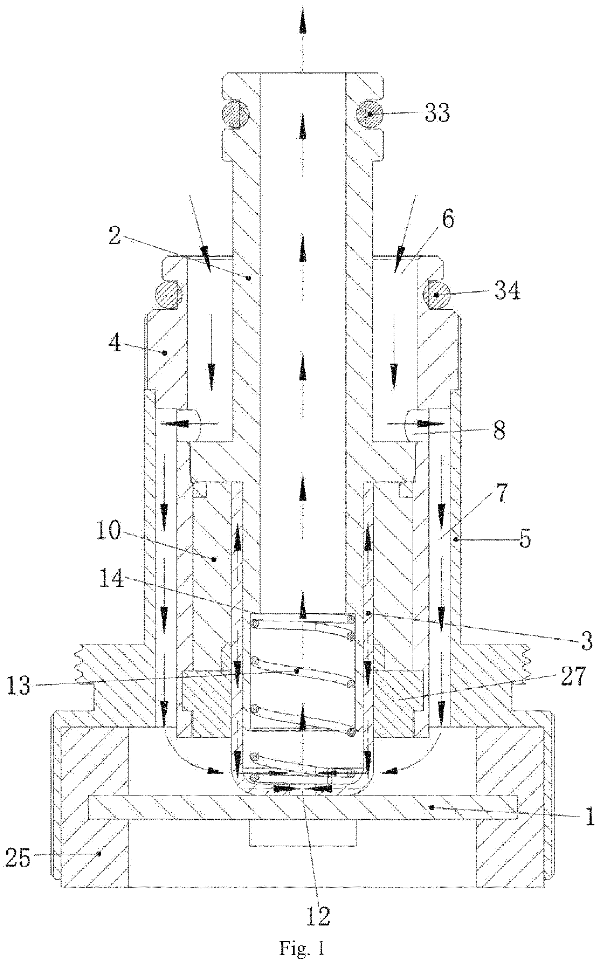

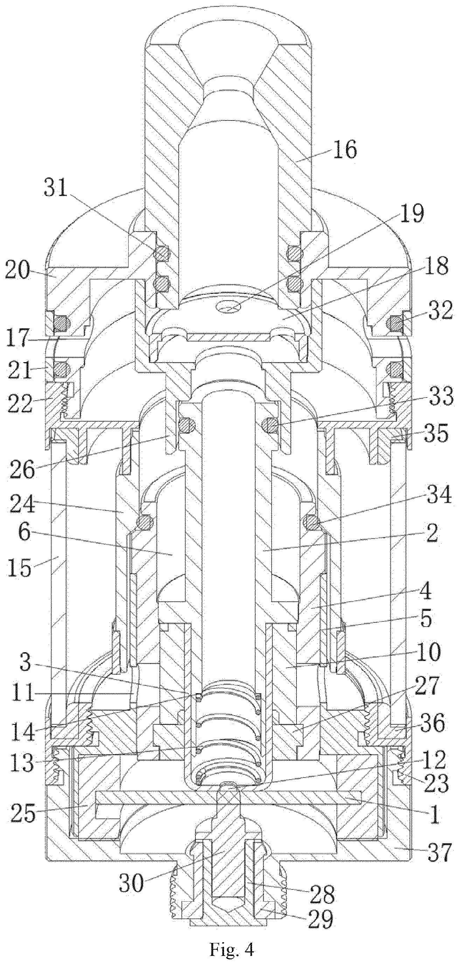

As shown in Fig.1 to Fig.4, embodiment 1 of the ultrasonic electronic cigarette atomization core includes an atomization core sleeve, an atomization piece 1 is provided in the atomization core sleeve, an air passage, an air pass pipe 2 and a cup-shaped tobacco tar guide cotton 3 are further provided in the atomization core sleeve, an outer bottom surface of the tobacco tar guide cotton 3 is in contact with a top surface of the atomization piece 1, and a side wall of the tobacco tar guide cotton 3 is sheathed at the outside of the bottom of the side wall of the air pass pipe 2; and the airflow enters from the air passage, flows by an inner bottom surface of the tobacco tar guide cotton 3 after penetrating through the bottom of the side wall of the tobacco tar guide cotton 3, and outflows from the air pass pipe 2.

The atomization core sleeve includes an atomization core outer sleeve 5 and an atomization core inner sleeve 4 sheathed in the atomization core outer sleeve 5, a gap 6 is provided between the top end of the inner side wall of the atomization core inner sleeve 4 and an outer side wall of the air pass pipe 2, an air pass groove 7 which communicates with the bottom of the side wall of the tobacco tar guide cotton 3 is provided in a bottom end of the outer side wall of the atomization core inner sleeve 4, and a first through hole 8 which communicates the gap 6 with the air pass groove 7 is provided in the side wall of the atomization core inner sleeve 4. The direction of the arrows as shown in Fig.1 are the airflow flow direction.

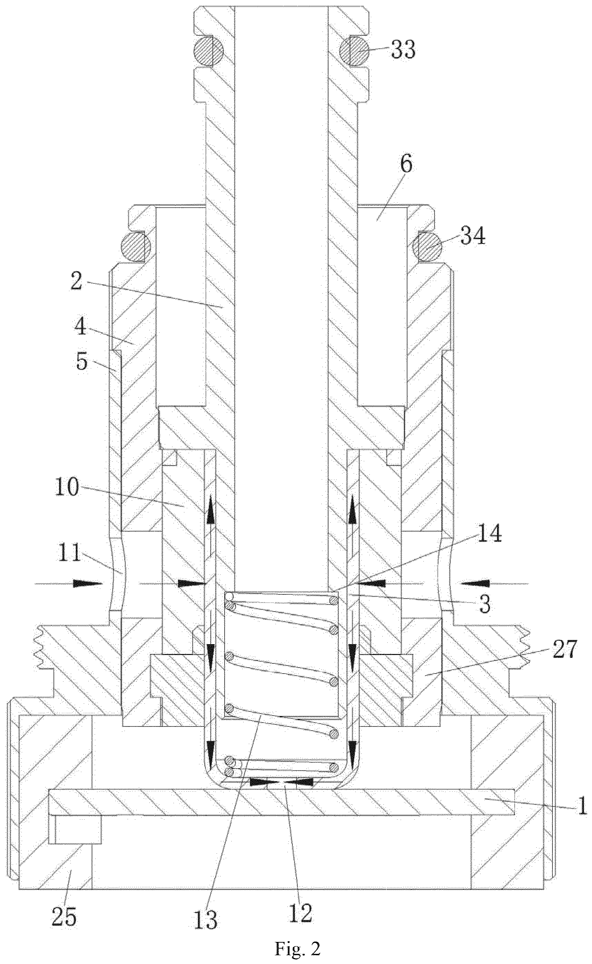

Tobacco tar storage cotton 10 sheathed at the outside of the side wall of the tobacco tar guide cotton 3 is further provided in the atomization core sleeve, and a tobacco tar inlet hole 11 which injects tobacco tar to the tobacco tar storage cotton 10 is provided in the side wall of the atomization core sleeve (including the atomization core inner sleeve 4 and the atomization core outer sleeve 5). The direction of the arrows as shown in Fig.2 are the tobacco tar guide direction.

A silica gel base 25 is provided at the bottom of the atomization core outer sleeve 5, and the atomization piece 1 is erected on the silica gel base 25.

A third through hole 12 corresponding to the central position of the atomization piece 1 is provided in the tobacco tar guide cotton 3.

An elastic bracket 13 is further provided in the air pass pipe 2, and a step part 14 is provided on the inner side wall of the air pass pipe 2; and the bottom end of the elastic bracket 13 props against the inner bottom surface of the tobacco tar guide cotton 3, and the top end of the elastic bracket 13 props against the step part 14. The elastic bracket 13 is a spring.

An ultrasonic electronic cigarette atomizer includes a tobacco tar bin 15 and a suction nozzle 16 located at the top of the tobacco tar bin 15, the ultrasonic electronic cigarette atomization core in Fig.1 and Fig.2 is provided in the tobacco tar bin 15, the ultrasonic electronic cigarette atomizer further includes a first air inlet hole 17 which communicates with the air inlet passage, and the top end of the air pass pipe 2 communicates with the suction nozzle 16.

An airflow baffle 18 vertical to the axial line of the air pass pipe 2 is further provided above the air pass pipe 2, the air pass pipe 2 aligns to the central position of the airflow baffle 18, a fourth through hole 19 deviated from the central position of the airflow baffle 18 is provided in the airflow baffle 18, and the air pass pipe 2 communicates with the suction nozzle 16 through the third through hole 12.

The air pass pipe 2 is connected with a suction nozzle base 20 through a connecting joint 26, the airflow baffle 18 is provided in the connecting joint 26, and the bottom of the side wall of the connecting joint 26 is sheathed at the outside of the top of the side wall of the air pass pipe 2.

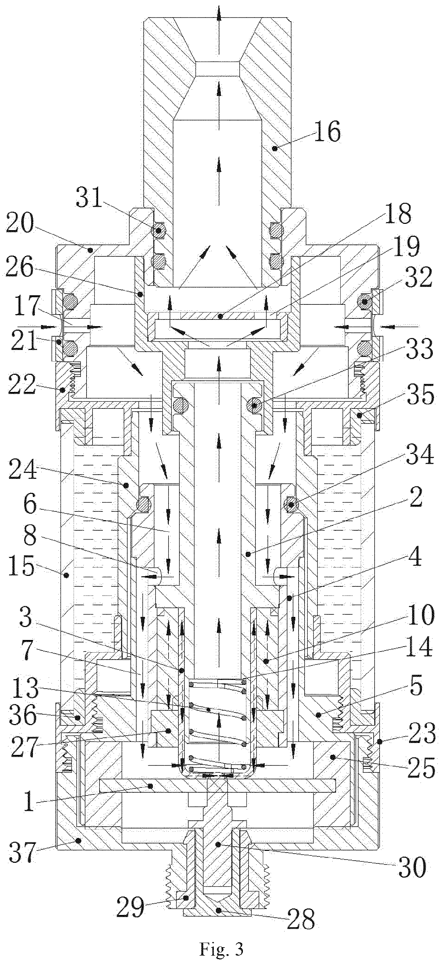

The atomizer further includes a suction nozzle base 20 which connects the side wall of the tobacco tar bin 15 with the suction nozzle 16, the first air inlet hole 17 is provided in the side wall of the suction nozzle base 20, and an air adjusting ring 21 whose position is corresponding to that of the first air inlet hole 17 is further provided at the outside of the side wall of the suction nozzle base 20. The direction of the arrows as shown in Fig.3 are the airflow flow direction.

The atomizer further includes a base 37; an upper end cover 22 is connected between the top of the side wall of the tobacco tar bin 15 and the side wall of the suction nozzle base 20, a lower end cover 23 is connected to the bottom of the side wall of the tobacco tar bin 15, the top of the atomization core sleeve is connected with the upper end cover 22 through a connecting pipe 24, and the bottom of the atomization core sleeve is in threaded connection with the top of the side wall of the lower end cover 23; and the side wall of the base 37 is in threaded connection with the bottom of the side wall of the lower end cover 23.

An electrode ring 28 is provided at the bottom of the base 37, an insulating ring 29 is provided between the electrode ring 28 and the inner wall of the base 37, the bottom of a spring plunger 30 stretches into the electrode ring 28, and the top of the spring plunger 30 props against the bottom surface of the atomization piece 1.

In order to prevent air leakage or tobacco tar leakage, a first sealing ring 31 is provided between the suction nozzle 16 and the inner side wall of the suction nozzle base 20, a second sealing ring 32 is provided between the outer side wall of the suction nozzle base 20 and the air adjusting ring 21, a third sealing ring 33 is provided between the outer side wall of the air pass pipe 2 and the connecting joint 26, a fourth sealing ring 34 is provided between the atomization core sleeve and the inner side wall of the connecting pipe 24, an upper sealing ring 35 is provided between the top of the side wall of the tobacco tar bin 15 and the upper end cover 22, a lower sealing ring 36 is provided between the bottom of the side wall of the tobacco tar bin 15 and the lower end cover 23, and a silica gel plug 27 sheathed between the atomization core inner sleeve and the side wall of the tobacco tar guide cotton 3 is provided at the bottom of the tobacco tar storage cotton 10.

### Embodiment 2

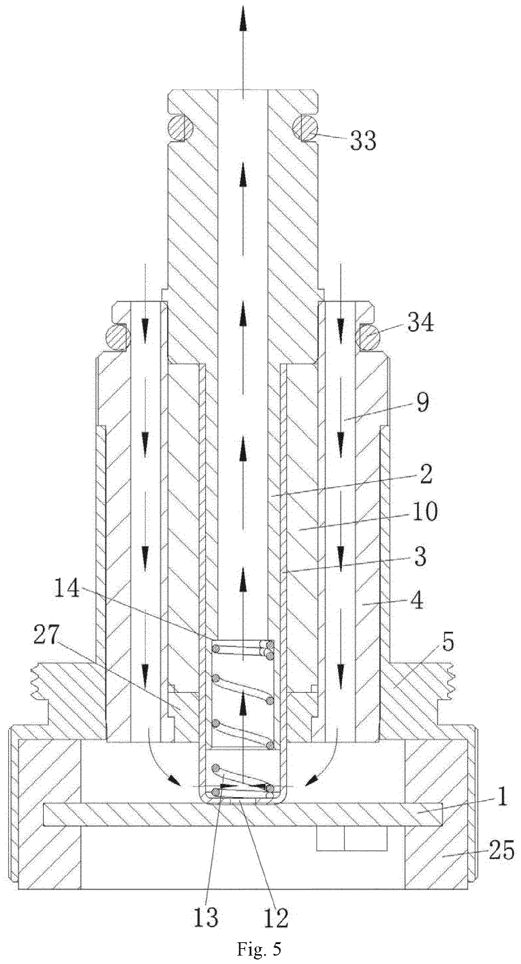

As shown in Fig.5 to Fig.8, embodiment 2 of the ultrasonic electronic cigarette atomization core includes an atomization core sleeve, an atomization piece 1 is provided in the atomization core sleeve, an air passage, an air pass pipe 2 and a cup-shaped tobacco tar guide cotton 3 are further provided in the atomization core sleeve, an outer bottom surface of the tobacco tar guide cotton 3 is in contact with a top surface of the atomization piece 1, and a side wall of the tobacco tar guide cotton 3 is sheathed at the outside of the bottom of the side wall of the air pass pipe 2; and the airflow enters from the air passage, flows by an inner bottom surface of the tobacco tar guide cotton 3 after penetrating through the bottom of the side wall of the tobacco tar guide cotton 3, and outflows from the air pass pipe 2.

The atomization core sleeve includes an atomization core outer sleeve 5 and an atomization core inner sleeve 4 sheathed in the atomization core outer sleeve 5, and a second through hole 9 which is located in the above-below direction and communicates with the bottom of the side wall of the tobacco tar guide cotton 3 is provided in the side wall of the atomization core inner sleeve 4. The direction of the arrows as shown in Fig.5 are the airflow flow direction.

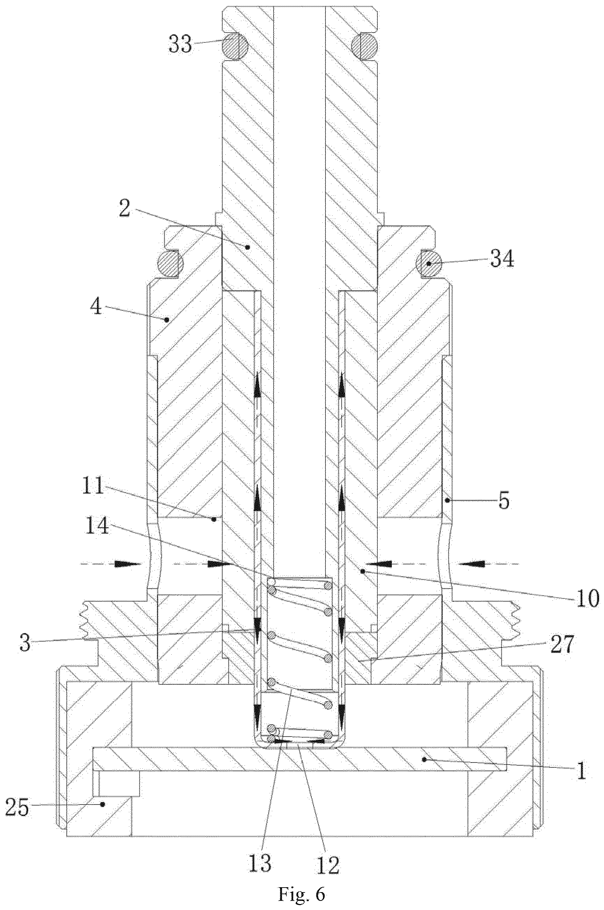

Tobacco tar storage cotton 10 sheathed at the outside of the side wall of the tobacco tar guide cotton 3 is further provided in the atomization core sleeve, and a tobacco tar inlet hole 11 which injects tobacco tar to the tobacco tar storage cotton 10 is provided in the side wall of the atomization core sleeve (including the atomization core inner sleeve 4 and the atomization core outer sleeve 5). The direction of arrows as shown in Fig.6 are the tobacco tar guide direction.

A silica gel base 25 is provided at the bottom of the atomization core outer sleeve 5, and the atomization piece 1 is erected on the silica gel base 25.

A third through hole 12 corresponding to the central position of the atomization piece 1 is provided in the tobacco tar guide cotton 3.

An elastic bracket 13 is further provided in the air pass pipe 2, and a step part 14 is provided on the inner side wall of the air pass pipe 2; and the bottom end of the elastic bracket 13 props against the inner bottom surface of the tobacco tar guide cotton 3, and the top end of the elastic bracket 13 props against the step part 14. The elastic bracket 13 is a spring.

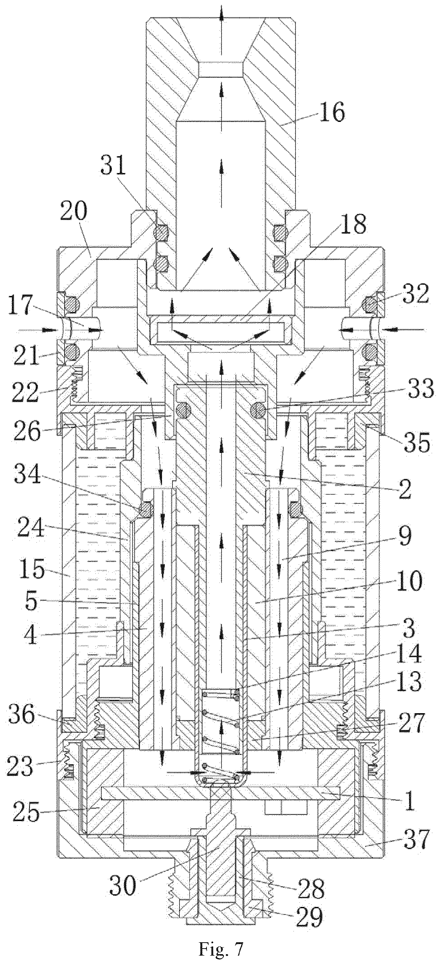

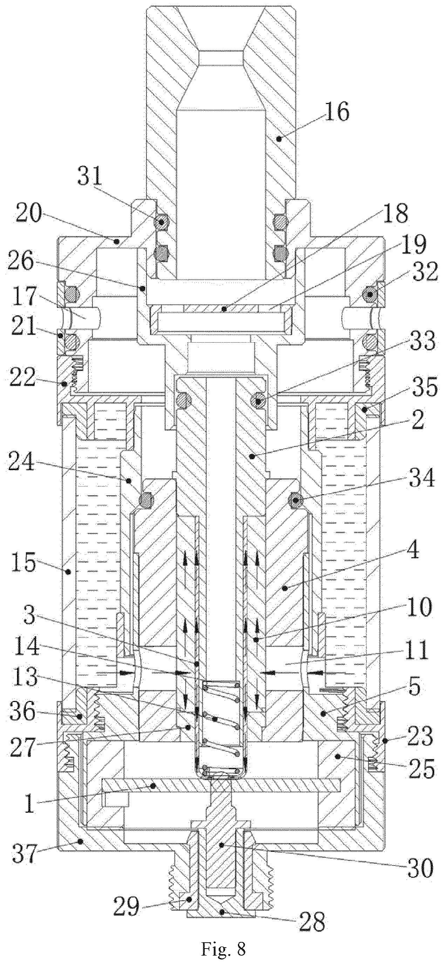

The ultrasonic electronic cigarette atomization core in Fig.5 and Fig.6 is provided in the tobacco tar bin 15 of the ultrasonic electronic cigarette atomizer in embodiment 2, excluding the structure of the atomization core, the other structures of the atomizer are the same as those in embodiment 1, and will not be repeated redundantly herein, but it does not affect those skilled in the art to understand and implement the present invention. The direction of the arrows as shown in Fig.7 are the airflow flow direction. The direction of the arrows as shown in Fig.8 are the tobacco tar guide direction.

### Embodiment 3

As shown in Fig.1 to Fig.8, embodiment 3 of the ultrasonic electronic cigarette atomization core includes an atomization core sleeve, an atomization piece 1 and tobacco tar guide cotton 3 whose bottom surface props against a top surface of the atomization piece 1 are provided in the atomization core sleeve, a third through hole 12 corresponding to the central position of the atomization piece 1 is provided in the tobacco tar guide cotton 3, an air passage and an air pass pipe 2 are further provided in the atomization core sleeve, and a bottom end of the air pass pipe 2 aligns to the third through hole 12; and the airflow enters from a top end of the air pass pipe 2, flows by the bottom end of the air pass pipe 2, the third through hole 12 and an upper surface of the atomization piece 1 in sequence, then penetrates through the bottom of the side wall of the tobacco tar guide cotton 3, and outflows from the air passage.

The atomization core sleeve includes an atomization core outer sleeve 5 and an atomization core inner sleeve 4 sheathed in the atomization core outer sleeve 5, an air pass groove 7 which communicates with the third through hole 12 is provided in the bottom end of the outer side wall of the atomization core inner sleeve 4, a gap 6 is provided between the top end of the inner side wall of the atomization core inner sleeve 4 and an outer side wall of the air pass pipe 2, and a first through hole 8 which communicates the air pass groove 7 with the gap 6 is provided in the side wall of the atomization core inner sleeve 4. The direction of arrows as shown in Fig. 1 are the airflow flow direction.

The tobacco tar guide cotton 3 is of a cup-shaped structure, the side wall of the tobacco tar guide cotton 3 is sheathed at the outside of the side wall of the air pass pipe 2, tobacco tar storage cotton 10 sheathed at the outside of the side wall of the tobacco tar guide cotton 3 is further provided in the atomization core sleeve, a tobacco tar inlet hole 11 which injects tobacco tar to the tobacco tar storage cotton 10 is provided in the side wall of the atomization core sleeve (including the atomization core inner sleeve 4 and the atomization core outer sleeve 5), and the tobacco tar in the tobacco tar bin 15 enters the atomization core through the tobacco tar inlet hole 11. A silica gel base 25 is provided at the bottom of the atomization core outer sleeve 5, and the atomization piece 1 is erected on the silica gel base 25.

An elastic bracket 13 is further provided in the air pass pipe 2, and a step part 14 is provided on the inner side wall of the air pass pipe 2; and the bottom end of the elastic bracket 13 props against the inner bottom surface of the tobacco tar guide cotton 3, and the top end of the elastic bracket 13 props against the step part 14. The elastic bracket 13 is a spring.

An ultrasonic electronic cigarette atomizer includes a tobacco tar bin 15 and a suction nozzle 16 located at the top of the tobacco tar bin 15, the ultrasonic electronic cigarette atomization core in Fig.1 is provided in the tobacco tar bin 15, the ultrasonic electronic cigarette atomizer further includes an air inlet passage which communicates with the air pass pipe 2, and an air outlet hole 38 which communicates the suction nozzle 16 with an air outlet passage.

The atomizer further includes a suction nozzle base 20 which connects the side wall of the tobacco tar bin 15 with the suction nozzle 16, an air inlet ring 39 is provided in the suction nozzle base 20, a first air inlet hole 17 is provided in the side wall of the suction nozzle base 20, a second air inlet hole 40 which communicates the first air inlet hole 17 with the air pass pipe 2 is provided in the air inlet ring 39, and the air outlet hole 38 is provided in the air inlet ring 39.

An air adjusting ring 21 whose position is corresponding to that of the first air inlet hole 17 is further provided on the side wall of the suction nozzle base 20.

The directions of solid arrows as shown in Fig.2 and Fig.3 are the airflow flow directions, and the direction of dashed arrows as shown in Fig.2 are the tobacco tar guide direction.

An upper end cover 22 is connected between the top of the side wall of the tobacco tar bin 15 and the side wall of the suction nozzle base 20, a lower end cover 23 is connected to the bottom of the side wall of the tobacco tar bin 15, the top of the atomization core sleeve is connected with the upper end cover 22 through a connecting pipe 24, and the bottom of the atomization core sleeve is in threaded connection with the top of the side wall of the lower end cover 23.

The atomizer further includes a base 37, and the side wall of the base 37 is in threaded connection with the bottom of the side wall of the lower end cover 23. An electrode ring 28 is provided at the bottom of the base 37, an insulating ring 29 is provided between the electrode ring 28 and the inner wall of the base 37, the bottom of a spring plunger 30 stretches into the electrode ring 28, and the top of the spring plunger 30 props against the bottom surface of the atomization piece 1.

In order to prevent air leakage or tobacco tar leakage, a first sealing ring 31 is provided between the suction nozzle 16 and the inner side wall of the suction nozzle base 20, a second sealing ring 32 is provided between the outer side wall of the suction nozzle base 20 and the air adjusting ring 21, a third sealing ring 33 is provided between the outer side wall of the air pass pipe 2 and the air inlet ring 39, a fourth sealing ring 34 is provided between the atomization core sleeve and the inner side wall of the connecting pipe 24, an upper sealing ring 35 is provided between the top of the side wall of the tobacco tar bin 15 and the upper end cover 22, a lower sealing ring 36 is provided between the bottom of the side wall of the tobacco tar bin 15 and the lower end cover 23, and a silica gel plug 27 sheathed between the atomization core inner sleeve 4 and the side wall of the tobacco tar guide cotton 3 is provided at the bottom of the tobacco tar storage cotton 10.

In an explosive view, the first sealing ring 31, the second sealing ring 32, the third sealing ring 33 and the fourth sealing ring 34 are not shown, but it does not affect those skilled in the art to understand and implement the present invention.

### Embodiment 4

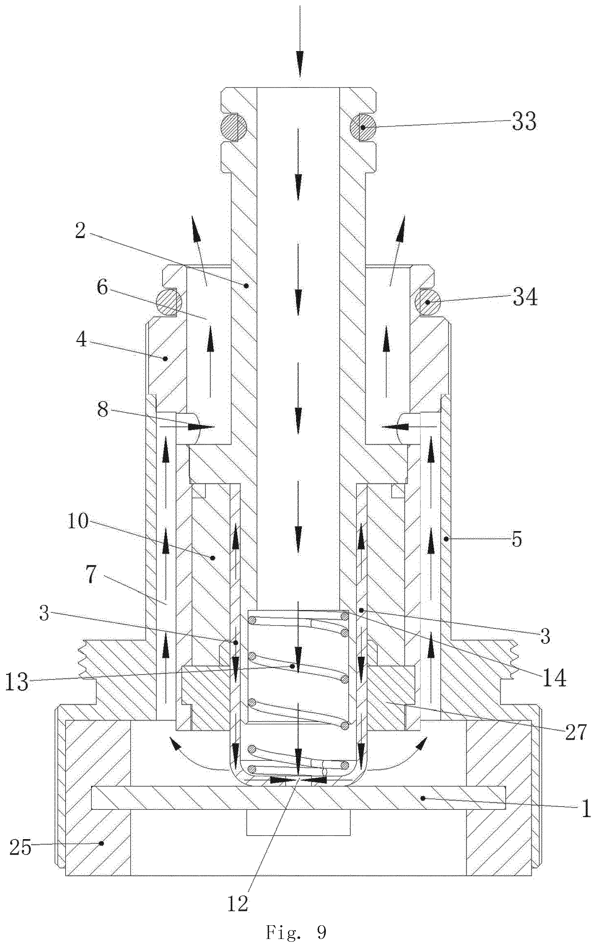

As shown in Fig.9 to Fig.11, embodiment 4 of the ultrasonic electronic cigarette atomization core includes an atomization core sleeve, an atomization piece 1 and tobacco tar guide cotton 3 whose bottom surface props against a top surface of the atomization piece 1 are provided in the atomization core sleeve, a third through hole 12 corresponding to the central position of the atomization piece 1 is provided in the tobacco tar guide cotton 3, an air passage and an air pass pipe 2 are further provided in the atomization core sleeve, and a bottom end of the air pass pipe 2 aligns to the third through hole 12; and the airflow enters from a top end of the air pass pipe 2, flows by the bottom end of the air pass pipe 2, the third through hole 12 and an upper surface of the atomization piece 1 in sequence, then penetrates through the bottom of the side wall of the tobacco tar guide cotton 3, and outflows from the air passage.

The atomization core sleeve includes an atomization core outer sleeve 5 and an atomization core inner sleeve 4 sheathed in the atomization core outer sleeve 5, and a second through hole 9 which is located in the above-below direction and communicates with the third through hole 12 is provided in the side wall of the atomization core inner sleeve 4. The direction of solid arrows as shown in Fig.9 are the airflow flow direction. The tobacco tar guide cotton 3 is of a cup-shaped structure, the side wall of the tobacco tar guide cotton 3 is sheathed at the outside of the side wall of the air pass pipe 2, tobacco tar storage cotton 10 sheathed at the outside of the side wall of the tobacco tar guide cotton 3 is further provided in the atomization core sleeve, and a tobacco tar inlet hole 11 which injects tobacco tar to the tobacco tar storage cotton 10 is provided in the side wall of the atomization core sleeve (including the atomization core inner sleeve 4 and the atomization core outer sleeve 5). A silica gel base 25 is provided at the bottom of the atomization core outer sleeve 5, and the atomization piece 1 is erected on the silica gel base 25. The direction of dashed arrows as shown in Fig.9 are the tobacco tar guide direction.

An elastic bracket 13 is further provided in the air pass pipe 2, and a step part 14 is provided on the inner side wall of the air pass pipe 2; and the bottom end of the elastic bracket 13 props against the inner bottom surface of the tobacco tar guide cotton 3, and the top end of the elastic bracket 13 props against the step part 14.

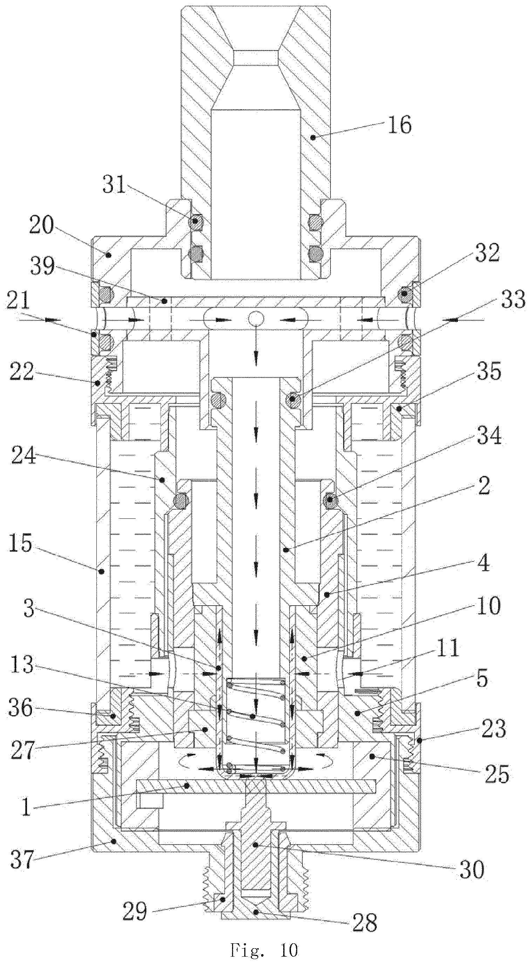

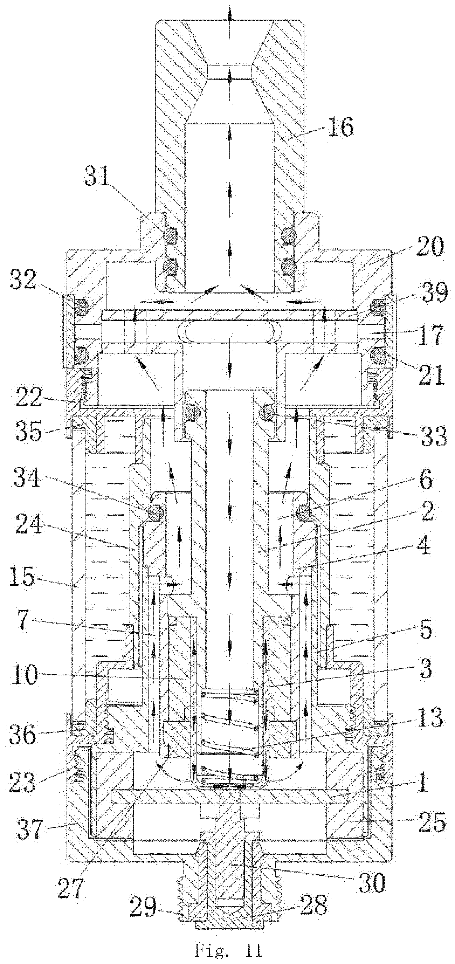

The ultrasonic electronic cigarette atomization core in Fig.9 is provided in the tobacco tar bin 15 of the ultrasonic electronic cigarette atomizer in embodiment 4, excluding the structure of the atomization core, the other structures of the atomizer are the same as those in embodiment 3, and will not be repeated redundantly herein, but it does not affect those skilled in the art to understand and implement the present invention. The directions of the arrows as shown in Fig.10 and Fig.11 are the airflow flow directions. Although the embodiments of the present invention have been described in combination with the accompanying drawings, the present invention is not limited to the specific embodiments described above which are merely illustrative but not restrictive, those of ordinary people skilled in the art can also make various forms without departing from the scope of protection of the invention as defined by the appended claims.

## Claims

1. An ultrasonic electronic cigarette atomization core, comprising an atomization core sleeve, an atomization piece (1) is provided in the atomization core sleeve, wherein an air passage, an air pass pipe (2) and a cup-shaped tobacco tar guide cotton (3) are further provided in the atomization core sleeve, an outer bottom surface of the tobacco tar guide cotton (3) is in contact with a top surface of the atomization piece (1), and a side wall of the tobacco tar guide cotton (3) is sheathed at the outside of the bottom of the side wall of the air pass pipe (2); and
the airflow enters from the air passage, flows by an inner bottom surface of the tobacco tar guide cotton (3) after penetrating through the bottom of the side wall of the tobacco tar guide cotton (3), and outflows from the air pass pipe (2); or the airflow enters from a top end of the air pass pipe (2), penetrates through the bottom of the side wall of the tobacco tar guide cotton (3) after flowing by the tobacco tar guide cotton (3), and outflows from the air passage.

2. The ultrasonic electronic cigarette atomization core of claim 1, wherein the atomization core sleeve comprises an atomization core outer sleeve (5) and an atomization core inner sleeve (4) sheathed in the atomization core outer sleeve (5), a gap (6) is provided between the top end of an inner side wall of the atomization core inner sleeve (4) and an outer side wall of the air pass pipe (2), an air pass groove (7) which communicates with the bottom of the side wall of the tobacco tar guide cotton (3) is provided in a bottom end of the outer side wall of the atomization core inner sleeve (4), and a first through hole (8) which communicates the gap (6) with the air pass groove (7) is provided in the side wall of the atomization core inner sleeve (4).

3. The ultrasonic electronic cigarette atomization core of claim 1, wherein the atomization core sleeve comprises an atomization core outer sleeve (5) and an atomization core inner sleeve (4) sheathed in the atomization core outer sleeve (5), and a second through hole (9) which is located in the above-below direction and communicates with the bottom of the side wall of the tobacco tar guide cotton (3) is provided in the side wall of the atomization core inner sleeve (4).

4. The ultrasonic electronic cigarette atomization core of any one of claims 1-3, wherein tobacco tar storage cotton (10) sheathed at the outside of the side wall of the tobacco tar guide cotton (3) is further provided in the atomization core sleeve, and a tobacco tar inlet hole (11) which injects tobacco tar to the tobacco tar storage cotton (10) is provided in the side wall of the atomization core sleeve.

5. The ultrasonic electronic cigarette atomization core of any one of claims 1-3, wherein a third through hole (12) corresponding to the central position of the atomization piece (1) is provided in the tobacco tar guide cotton (3).

6. The ultrasonic electronic cigarette atomization core of any one of claims 1-3, wherein the ultrasonic electronic cigarette atomization core further comprises an elastic bracket (13), and a step part (14) is provided on the inner side wall of the air pass pipe (2); and the bottom end of the elastic bracket (13) props against the inner bottom surface of the tobacco tar guide cotton (3), and the top end of the elastic bracket (13) props against the step part (14).

7. An ultrasonic electronic cigarette atomizer, comprising a tobacco tar bin (15), wherein the ultrasonic electronic cigarette atomization core of any one of claims 1-6 is provided in the tobacco tar bin (15).

8. The ultrasonic electronic cigarette atomizer of claim 7, wherein the ultrasonic electronic cigarette atomizer further comprises a suction nozzle (16) located at the top of the tobacco tar bin (15), and a first air inlet hole (17) which communicates with the air passage, and the top end of the air pass pipe (2) communicates with the suction nozzle (16).

9. The ultrasonic electronic cigarette atomizer of claim 8, wherein an airflow baffle (18) vertical to the axial line of the air pass pipe (2) is further provided above the air pass pipe (2), the air pass pipe (2) aligns to the central position of the airflow baffle (18), a fourth through hole (19) deviated from the central position of the airflow baffle (18) is provided in the airflow baffle (18), and the air pass pipe (2) communicates with the suction nozzle (16) through the third through hole (12).

10. The ultrasonic electronic cigarette atomizer of claim 8, wherein the ultrasonic electronic cigarette atomizer further comprises a suction nozzle base (20) which connects the side wall of the tobacco tar bin (15) with the suction nozzle (16), the first air inlet hole (17) is provided in the side wall of the suction nozzle base (20), and an air adjusting ring (21) corresponding to the first air inlet hole (17) in position is further provided at the outside of the side wall of the suction nozzle base (20).

11. The ultrasonic electronic cigarette atomizer of claim 7, wherein the ultrasonic electronic cigarette atomizer further comprises a suction nozzle (16) located at the top of the tobacco tar bin (15), an air inlet passage which communicates with the air pass pipe (2), and an air outlet hole (38) which communicates the suction nozzle (16) with the air passage.

12. The ultrasonic electronic cigarette atomizer of claim 11, wherein the ultrasonic electronic cigarette atomizer further comprises a suction nozzle base (20) which connects the side wall of the tobacco tar bin (15) with the suction nozzle (16), an air inlet ring (39) is provided in the suction nozzle base (20), the first air inlet hole (17) is provided in the side wall of the suction nozzle base (20), a second air inlet hole (40) which communicates the first air inlet hole (17) with the air pass pipe (2) is provided in the air inlet ring (39), and the air outlet hole (38) is provided in the air inlet ring (39).

13. The ultrasonic electronic cigarette atomizer of claim 12, wherein an air adjusting ring (21) corresponding to the first air inlet hole (17) in position is further provided on the side wall of the suction nozzle base (20).

14. The ultrasonic electronic cigarette atomizer of claim 10, 12 or 13, wherein the ultrasonic electronic cigarette atomizer further comprises a base (37); an upper end cover (22) is connected between the top of the side wall of the tobacco tar bin (15) and the side wall of the suction nozzle base (20), a lower end cover (23) is connected to the bottom of the side wall of the tobacco tar bin (15), the top of the atomization core sleeve is connected with the upper end cover (22) through a connecting pipe (24), and the bottom of the atomization core sleeve is in threaded connection with the top of the side wall of the lower end cover (23); and the side wall of the base (37) is in threaded connection with the bottom of the side wall of the lower end cover (23).

## Patentansprüche

1. Ultraschallzerstäubungskern für eine elektronische Zigarette, mit einer Zerstäubungskernhülse, bei dem ein Zerstäubungsstück (1), das in der Zerstäubungskernhülse vorgesehen ist, bei dem eine Luftpassage, ein Luftpassagenrohr (2) und eine becherförmige Tabakteerführungswatte (3) weiter in der Zerstäubungskernhülse vorgesehen sind, eine äußere Bodenoberfläche der Tabakteerführungswatte (3) in Kontakt mit einer oberen Oberfläche des Zerstäubungsstücks (1) ist, und eine Seitenwand der Tabakteerführungswatte (3) an der Außenseite des Bodens der Seitenwand des Luftpassagenrohrs (2) ummantelt ist; und
die Luftströmung von der Luftpassage eintritt, durch eine innere Bodenoberfläche der Tabakteerführungswatte (3) nach Durchdringen durch den Boden der Seitenwand der Tabakteerführungswatte (3) strömt, und aus dem Luftpassagenrohr (2) herausströmt, oder die Luftströmung von einem oberen Ende des Luftpassagenrohrs (2) eintritt, durch den Boden der Seitenwand der Tabakteerführungswatte (3) nach Strömen durch die Tabakteerführungswatte (3) durchdringt und von der Luftpassage herausströmt.

2. Ultraschallzerstäubungskern für eine elektronische Zigarette nach Anspruch 1, bei dem die Zerstäubungskernhülse eine Zerstäubungskernaußenhülse (5) und eine Zerstäubungskerninnenhülse (4) aufweist, die in der Zerstäubungskernaußenhülse (5) ummantelt ist, einen Spalt (6) zwischen dem oberen Ende einer Innenseitenwand der Zerstäubungskerninnenhülse (4) und einer Außenseitenwand des Luftpassagenrohrs (2) vorgesehen ist, eine Luftpassagennut (7), welche mit dem Boden der Seitenwand der Tabakteerführungswatte (3) kommunizierend in Verbindung steht, in einem Bodenende der Außenseitenwand der Zerstäubungskerninnenhülse (4) vorgesehen ist, und ein erstes Durchgangsloch (8), welches den Spalt (6) mit der Luftpassagennut (7) kommunizierend verbindet, in der Seitenwand der Zerstäubungskerninnenhülse (4) vorgesehen ist.

3. Ultraschallzerstäubungskern für eine elektronische Zigarette nach Anspruch 1, bei dem die Zerstäubungskernhülse eine Zerstäubungskernaußenhülse (5) und eine Zerstäubungskerninnenhülse (4) aufweist, die in der Zerstäubungskernaußenhülse (5) ummantelt ist, und ein zweites Durchgangsloch (9), welches sich in der Oben-Unten-Richtung befindet und mit dem Boden der Seitenwand der Tabakteerführungswatte (3) kommunizierend in Verbindung steht, in der Seitenwand der Zerstäubungskerninnenhülse (4) vorgesehen ist.

4. Ultraschallzerstäubungskern für eine elektronische Zigarette nach einem der Ansprüche 1 bis 3, bei dem eine Tabakteerspeicherwatte (10), die an der Außenseite der Seitenwand der Tabakteerführungswatte (3) ummantelt ist, ferner in der Zerstäubungskernhülse vorgesehen ist, und ein Tabakteereinlassloch (11), welches Tabakteer zu der Tabakteerspeicherwatte (10) ausstößt, in der Seitenwand der Zerstäubungskernhülse vorgesehen ist.

5. Ultraschallzerstäubungskern für eine elektronische Zigarette nach einem der Ansprüche 1 bis 3, bei dem ein drittes Durchgangsloch (12) entsprechend zu der mittleren Position des Zerstäubungsstückes (1) bei der Tabakteerführungswatte (3) vorgesehen ist.

6. Ultraschallzerstäubungskern für eine elektronische Zigarette nach einem der Ansprüche 1 bis 3, bei dem der Ultraschallzerstäubungskern der elektronischen Zigarette ferner eine elastische Halterung (13) aufweist, und ein Stufenteil (14) an der Innenseitenwand des Luftpassagenrohrs (2) vorgesehen ist; und das Bodenende der elastischen Halterung (13) sich gegen die Innenbodenoberfläche der Tabakteerführungswatte (3) stützt, und das obere Ende der elastischen Halterung (13) sich gegen den Stufenteil (14) stützt.

7. Ultraschallzerstäuber für eine elektronische Zigarette, mit einem Tabakteerbehälter (15), bei dem der Ultraschallzerstäubungskern für eine elektronische Zigarette nach einem der Ansprüche 1 bis 6 in dem Tabakteerbehälter (15) vorgesehen ist.

8. Ultraschallzerstäuber für eine elektronische Zigarette nach Anspruch 7, bei dem der Ultraschallzerstäuber für eine elektronische Zigarette ferner einen Ansaugstutzen (16), der sich an der Oberseite des Tabakteerbehälters (15) befindet, und ein erstes Lufteinlassloch (17) aufweist, welches mit der Luftpassage kommunizierend in Verbindung steht, und das obere Ende des Luftpassagenrohrs (2) mit dem Ansaugstutzen (16) kommunizierend in Verbindung steht.

9. Ultraschallzerstäuber für eine elektronische Zigarette nach Anspruch 8, bei dem eine Luftströmungsablenkplatte (18) vertikal zu der axialen Linie des Luftpassagenrohrs (2) weiter oberhalb des Luftpassagenrohrs (2) vorgesehen ist, das Luftpassagenrohr (2) auf die mittlere Position der Luftströmungsablenkplatte (18) ausgerichtet ist, ein viertes Durchgangsloch (19), welches von der mittleren Position der Luftströmungsablenkplatte (18) abweicht, in der Luftströmungsablenkplatte (18) vorgesehen ist, und das Luftpassagenrohr (2) mit dem Ansaugstutzen (16) über das dritte Durchgangsloch (12) kommunizierend in Verbindung steht.

10. Ultraschallzerstäuber für eine elektronische Zigarette nach Anspruch 8, bei dem der Ultraschallzerstäuber für eine elektronische Zigarette ferner eine Ansaugstutzenbasis (20) aufweist,, welche die Seitenwand des Tabakteerbehälters (15) mit dem Ansaugstutzen (16) verbindet, das erste Lufteinlassloch (17) in der Seitenwand der Ansaugstutzenbasis (20) vorgesehen ist, und ferner weiter an der Außenseite der Seitenwand der Ansaugstutzenbasis (20) ein Luftjustierring (21) vorgesehen ist, der in Position dem ersten Lufteinlassloch (17) entspricht.

11. Ultraschallzerstäuber für eine elektronische Zigarette nach Anspruch 7, bei dem der Ultraschallzerstäuber für eine elektronische Zigarette ferner einen Ansaugstutzen (16), der sich an der Oberseite des Tabakteerbehälters (15) befindet, eine Lufteinlasspassage, welche mit dem Luftpassagenrohr (2) kommunizierend in Verbindung steht, und ein Luftauslassloch (38) aufweist, welches den Ansaugstutzen (16) kommunizierend mit der Luftpassage verbindet.

12. Ultraschallzerstäuber für eine elektronische Zigarette nach Anspruch 11, bei dem der Ultraschallzerstäuber für eine elektronische Zigarette ferner eine Ansaugstutzenbasis (20) aufweist, welche die Seitenwand des Tabakteerbehälters (15) mit dem Ansaugstutzen (16) verbindet, ein Lufteinlassring (39) an der Ansaugstutzenbasis (20) vorgesehen ist, das erste Lufteinlassloch (17) in der Seitenwand der Ansaugstutzenbasis (20) vorgesehen ist, ein zweites Lufteinlassloch (40), welches das erste Lufteinlassloch (17) mit dem Luftpassagenrohr (2) kommunizierend verbindet, in dem Lufteinlassring (39) vorgesehen ist, und das Luftauslassloch (38) in dem Lufteinlassring (39) vorgesehen ist.

13. Ultraschallzerstäuber für eine elektronische Zigarette nach Anspruch 12, bei dem ferner an der Seitenwand der Ansaugstutzenbasis (20) ein Luftjustierring (21) vorgesehen ist, der in Position dem ersten Lufteinlassloch (17) entspricht.

14. Ultraschallzerstäuber für eine elektronische Zigarette nach Anspruch 10, 12 oder 13, bei dem der Ultraschallzerstäuber für eine elektronische Zigarette ferner eine Basis (37) aufweist; eine obere Endabdeckung (22) zwischen der Oberseite der Seitenwand des Tabakteerbehälters (15) und der Seitenwand des Ansaugstutzenbasis (20) verbunden ist, eine untere Endabdeckung (23) mit dem Boden der Seitenwand des Tabakteerbehälters (15) verbunden ist, die Oberseite der Zerstäubungskernhülse mit der oberen Endabdeckung (22) über ein Verbindungsrohr (24) verbunden ist, und der Boden der Zerstäubungskernhülse in Gewindeverbindung mit der Oberseite der Seitenwand der unteren Endabdeckung (23) ist; und die Seitenwand der Basis (37) in Gewindeverbindung mit dem Boden der Seitenwand der unteren Endabdeckung (23) ist.

## Revendications

1. Noyau d'atomisation de cigarette électronique par ultrasons, comprenant un manchon de noyau d'atomisation, une pièce d'atomisation (1) est prévue dans le manchon de noyau d'atomisation, dans lequel un passage d'air, un tuyau de passage d'air (2) et un coton de guidage de goudron de tabac en forme de coupe (3) sont en outre prévus dans le manchon de noyau d'atomisation, une surface inférieure externe du coton de guidage de goudron de tabac (3) est en contact avec une surface supérieure de la pièce d'atomisation (1), et une paroi latérale du coton de guidage de goudron de tabac (3) est gainée à l'extérieur du fond de la paroi latérale du tuyau de passage d'air (2) ; et
l'écoulement d'air entre depuis le passage d'air, s'écoule par une surface inférieure interne du coton de guidage de goudron de tabac (3) après avoir pénétré à travers le fond de la paroi latérale du coton de guidage de goudron de tabac (3), et sort du tuyau de passage d'air (2) ; ou l'écoulement d'air entre depuis une extrémité supérieure du tuyau de passage d'air (2), pénètre à travers le fond de la paroi latérale du coton de guidage de goudron de tabac (3) après s'être écoulé par le coton de guidage de goudron de tabac (3), et sort du passage d'air.

2. Noyau d'atomisation de cigarette électronique par ultrasons selon la revendication 1, dans lequel le manchon de noyau d'atomisation comprend un manchon externe de noyau d'atomisation (5) et un manchon interne de noyau d'atomisation (4) gainé dans le manchon externe de noyau d'atomisation (5), un espace (6) est prévu entre l'extrémité supérieure d'une paroi latérale interne du manchon interne de noyau d'atomisation (4) et une paroi latérale externe du tuyau de passage d'air (2), une rainure de passage d'air (7) qui communique avec le fond de la paroi latérale du coton de guidage de goudron de tabac (3) est prévue dans une extrémité inférieure de la paroi latérale externe du manchon interne de noyau d'atomisation (4), et un premier trou traversant (8) qui fait communiquer l'espace (6) avec la rainure de passage d'air (7) est prévu dans la paroi latérale du manchon interne de noyau d'atomisation (4).

3. Noyau d'atomisation de cigarette électronique par ultrasons selon la revendication 1, dans lequel le manchon de noyau d'atomisation comprend un manchon externe de noyau d'atomisation (5) et un manchon interne de noyau d'atomisation (4) gainé dans le manchon externe de noyau d'atomisation (5), et un second trou traversant (9) qui est situé dans la direction dessus-dessous et communique avec le fond de la paroi latérale du coton de guidage de goudron de tabac (3) est prévu dans la paroi latérale du manchon interne de noyau d'atomisation (4).

4. Noyau d'atomisation de cigarette électronique par ultrasons selon l'une quelconque des revendications 1 à 3, dans lequel le coton de stockage de goudron de tabac (10) gainé à l'extérieur de la paroi latérale du coton de guidage de goudron de tabac (3) est prévu plus loin dans le manchon de noyau d'atomisation, et un trou d'entrée de goudron de tabac (11) qui injecte du goudron de tabac dans le coton de stockage de goudron de tabac (10) est prévu dans la paroi latérale du manchon de noyau d'atomisation.

5. Noyau d'atomisation de cigarette électronique par ultrasons selon l'une quelconque des revendications 1 à 3, dans lequel un troisième trou traversant (12) correspondant à la position centrale de la pièce d'atomisation (1) est prévu dans le coton de guidage de goudron de tabac (3).

6. Noyau d'atomisation de cigarette électronique par ultrasons selon l'une quelconque des revendications 1 à 3, dans lequel le noyau d'atomisation de cigarette électronique par ultrasons comprend en outre un support élastique (13), et une partie étagée (14) est prévue sur la paroi latérale interne du tuyau de passage d'air (2) ; et l'extrémité inférieure du support élastique (13) s'appuie contre la surface inférieure interne du coton de guidage de goudron de tabac (3), et l'extrémité supérieure du support élastique (13) s'appuie contre la partie étagée (14).

7. Atomiseur de cigarette électronique par ultrasons, comprenant un bac à goudron de tabac (15), dans lequel le noyau d'atomisation de cigarette électronique par ultrasons selon l'une quelconque des revendications 1 à 6 est prévu dans le bac à goudron de tabac (15).

8. Atomiseur de cigarette électronique par ultrasons selon la revendication 7, dans lequel l'atomiseur de cigarette électronique par ultrasons comprend en outre une buse d'aspiration (16) située au sommet du bac à goudron de tabac (15), et un premier trou d'entrée d'air (17) qui communique avec le passage d'air, et l'extrémité supérieure du tuyau de passage d'air (2) communique avec la buse d'aspiration (16).

9. Atomiseur de cigarette électronique par ultrasons selon la revendication 8, dans lequel un déflecteur d'écoulement d'air (18) vertical à la ligne axiale du tuyau de passage d'air (2) est prévu plus loin au-dessus du tuyau de passage d'air (2), le tuyau de passage d'air (2) s'aligne sur la position centrale du déflecteur d'écoulement d'air (18), un quatrième trou traversant (19) dévié de la position centrale du déflecteur d'écoulement d'air (18) est prévu dans le déflecteur d'écoulement d'air (18), et le tuyau de passage d'air (2) communique avec la buse d'aspiration (16) par le troisième trou traversant (12).

10. Atomiseur de cigarette électronique par ultrasons selon la revendication 8, dans lequel l'atomiseur de cigarette électronique par ultrasons comprend en outre une base de buse d'aspiration (20) qui relie la paroi latérale du bac à goudron de tabac (15) à la buse d'aspiration (16), le premier trou d'entrée d'air (17) est prévu dans la paroi latérale de la base de buse d'aspiration (20), et une bague de réglage d'air (21) correspondant au premier trou d'entrée d'air (17) en position est en outre prévue à l'extérieur de la paroi latérale de la base de buse d'aspiration (20).

11. Atomiseur de cigarette électronique par ultrasons selon la revendication 7, dans lequel l'atomiseur de cigarette électronique par ultrasons comprend en outre une buse d'aspiration (16) située au sommet du bac à goudron de tabac (15), un passage d'entrée d'air qui communique avec le tuyau de passage d'air (2), et un trou de sortie d'air (38) qui fait communiquer la buse d'aspiration (16) avec le passage d'air.

12. Atomiseur de cigarette électronique par ultrasons selon la revendication 11, dans lequel l'atomiseur de cigarette électronique par ultrasons comprend en outre une base de buse d'aspiration (20) qui relie la paroi latérale du bac à goudron de tabac (15) à la buse d'aspiration (16), une bague d'entrée d'air (39) est prévu dans la base de buse d'aspiration (20), le premier trou d'entrée d'air (17) est prévu dans la paroi latérale de la base de buse d'aspiration (20), un second trou d'entrée d'air (40) qui fait communiquer le premier trou d'entrée d'air (17) avec le tuyau de passage d'air (2) est prévu dans la bague d'entrée d'air (39), et le trou de sortie d'air (38) est prévu dans l'anneau d'entrée d'air (39).

13. Atomiseur de cigarette électronique par ultrasons selon la revendication 12, dans lequel une bague de réglage d'air (21) correspondant au premier trou d'entrée d'air (17) en position est en outre prévue sur la paroi latérale de la base de buse d'aspiration (20).

14. Atomiseur de cigarette électronique par ultrasons selon la revendication 10, 12 ou 13, dans lequel l'atomiseur de cigarette électronique par ultrasons comprend en outre une base (37) ; un couvercle d'extrémité supérieur (22) est connecté entre le haut de la paroi latérale du bac à goudron de tabac (15) et la paroi latérale de la base de buse d'aspiration (20), un couvercle d'extrémité inférieur (23) est connecté au fond de la paroi latérale du bac à goudron de tabac (15), le haut du manchon de noyau d'atomisation est connecté au couvercle d'extrémité supérieur (22) par un tuyau de connexion (24), et le fond du manchon de noyau d'atomisation est en connexion filetée avec le haut de la paroi latérale du couvercle d'extrémité inférieur (23) ; et la paroi latérale de la base (37) est en connexion filetée avec le fond de la paroi latérale du couvercle d'extrémité inférieure (23).
